# FASCICULE DE BREVET EUROPEEN

(11) **EP 2 859 344 B1**
(45) Date de publication et mention de la délivrance du brevet: **02.05.2018**
(21) Numéro de dépôt: 13744587.0
(22) Date de dépôt: 11.06.2013
(51) Int. Cl.: G01N 33/52, G01N 33/543, G01N 33/68, G01N 33/80, B01D 67/00, B01L 3/00

(54) **DISPOSITIF DE DIAGNOSTIC IN VITRO ET UTILISATIONS**
IN-VITRO-DIAGNOSEVORRICHTUNG UND VERWENDUNGEN DAVON
IN VITRO DIAGNOSIS DEVICE AND USES THEREOF

(30) Priorité: 11.06.2012 FR 1255452
(43) Date de publication de la demande: 15.04.2015
(73) Titulaire: Diagast, 59120 Loos (FR)
(72) Inventeur: CHAIBI, Najim, F-33140 Villenave d'Ornon (FR); MALGOURIES, Sylvain, 16100 Cognac (FR); LUCAS, Megumi, F-33650 Martillac (FR)
(74) Mandataire: Regimbeau
(86) Numéro de dépôt international: PCT/FR2013/051358
(87) Numéro de publication internationale: WO 2013/186482

(56) Documents cités:
- EP-A1- 0 367 468
- EP-A2- 0 408 378
- WO-A1-98/20348
- WO-A1-98/25758
- WO-A1-2007/064462
- WO-A1-2012/010666
- WO-A2-03/008933
- WO-A2-2010/056889
- FR-A1- 2 892 820
- FR-A1- 2 917 174
- US-A- 5 185 127
- US-A1- 2005 124 077

## Description

La présente invention est relative à un dispositif de diagnostic in vitro, à partir d'un échantillon de sang ou d'un de ses composants, pour détecter des réactions entre des antigènes érythrocytaires et des anticorps dirigés spécifiquement contre ces antigènes.

L'invention vise également les utilisations de ce dispositif pour l'identification et la détermination de groupes sanguins.

Les diagnostics immuno-hématologiques ont pour objectif de prévenir ou de diagnostiquer une attaque de globules rouges par des anticorps. Pour cela, il est nécessaire de disposer d'outils permettant de déterminer les antigènes présents à la surface des globules rouges, leur présence ou leur absence permettant de définir le groupe sanguin, mais également d'identifier si un sang contient un ou plusieurs anticorps dirigés contre les antigènes connus des globules rouges, la présence d'un anticorps signant la possibilité d'une incompatibilité.

Les techniques habituellement utilisées, consistent donc à rechercher et identifier la présence ou l'absence d'antigènes de groupe sanguin à la surface des érythrocytes et/ou à rechercher et identifier la présence ou l'absence d'anticorps anti-antigènes de groupe sanguin dans le plasma.

Par exemple, pour le système ABO, l'épreuve de Beth-Vincent permet de déterminer les antigènes portés par les globules rouges, et l'épreuve complémentaire de Simonin-Michon ou contre épreuve-sérique permet de déterminer les anticorps circulant dans le sérum.

Dans l'épreuve de Beth-Vincent, les globules rouges de l'individu sont mis en présence de réactifs d'anticorps de spécificité connue. Généralement ce test est rendu visible par observation d'agglutination des globules rouges lorsque les anticorps reconnaissent les antigènes érythrocytaires correspondants.

Dans l'épreuve de Simonin, le plasma de l'individu est mis en présence de globules rouges tests appartenant chacun à un groupe antigénique précis du système ABO. Il s'agit d'un test d'agglutination d'hématies tests avec le plasma de l'individu.

Pour la recherche d'anticorps dits irréguliers, il s'agit de détecter la présence ou l'absence dans le sang d'un individu d'immunoglobulines dirigées contre divers antigènes érythrocytaires. Dans le cas d'une recherche d'auto-anticorps, on cherche directement les anticorps déjà fixés in vivo chez l'individu dans le test direct. Dans le cas d'une recherche d'allo-anticorps, on cherche à mettre en évidence la fixation de ces immunoglobulines sur des globules rouges tests dont les antigènes sont connus, avec la technique de Coombs indirect.

Il existe un grand nombre de procédés et de dispositifs utilisés pour le phénotypage dans le domaine de l'immuno-hématologie (par exemple le procédé décrit dans la demande FR 2892820 qui consiste en l'utilisation de billes magnétiques), mais les techniques existantes de phénotypage de groupes sanguins présentent de nombreux inconvénients.

Les techniques en microplaque par exemple nécessitent une phase de centrifugation suivie d'une étape d'agitation. L'étape d'agitation est critique car les multiples réactions simultanément présentes sur le support n'ont pas la même cinétique de remise en suspension. On risque donc de défaire de faibles agglutinations sans arriver à resuspendre de fortes agglutinations. Elles doivent être réalisées sous contrôle visuel et il faut être particulièrement attentif aux phénomènes d'adhérence de certains réactifs.

De même, lors de la mise en oeuvre de techniques en filtration par gel-test, il existe également un risque de non détection de certaines agglutinations notamment lors de l'épreuve plasmatique du groupe ABO à cause de la dissociation par cisaillement des petits agglutinats lors de leur passage dans le gel.

En outre, toutes ces techniques présentent un inconvénient majeur du fait qu'elles nécessitent une étape de centrifugation pour décanter les globules rouges ou les faire passer à travers le gel, étape contraignante qui augmente fortement le temps et le coût d'analyse, et qui nécessite l'utilisation de centrifugeuses encombrantes et difficiles à manipuler.

On connaît également la méthode d'immunofiltration, telle que décrite par exemple dans la demande EP-2.167.967, qui consiste à capturer un analyte présent dans un échantillon lors de son passage à travers une membrane poreuse portant un élément de capture et à révéler sa présence par un élément de révélation. Ce type de dispositif est constitué d'une zone de dépôt de l'échantillon, d'une membrane poreuse hydrophile sur laquelle est déposé un réactif de capture et sous laquelle est disposée une membrane absorbante. La mise en oeuvre d'un tel test consiste à déposer un échantillon pur ou dilué dans la zone de dépôt d'échantillon, qui traverse la membrane poreuse pour finir dans la membrane absorbante. Au cours de cette traversée par capillarité dans la membrane poreuse, si l'analyte correspondant à l'élément de capture est présent dans l'échantillon, celui-ci est immobilisé par l'agent de capture. Dans un second temps, il est nécessaire de révéler la présence sur le spot de capture de l'analyte recherché à l'aide d'un agent de révélation capable de détecter la présence de l'analyte sur la zone de capture et qui porte un élément lui permettant d'être détecté visuellement (produit coloré) ou révélé par une méthode physique ou chimique.

Toutefois cette méthode présente des problèmes de sensibilité et de spécificité importants, car lorsque l'échantillon est déposé il diffuse et imbibe la membrane poreuse, et de nombreux analytes se perdent dans le volume mort de la membrane poreuse ou passent en dehors de la zone de capture. Il en est de même pour la solution de révélation. Il est donc nécessaire de surdimensionner le système d'absorption pour pouvoir déposer des volumes plus importants d'échantillons à tester et de solution de révélation, ce qui empêche la réalisation de tests miniaturisables dont on contrôle la cinétique, exempts de remonté d'agents de révélation et utilisables sous un robot pipeteur.

Pour tenter de résoudre ce problème et concentrer le signal, il a été proposé dans la demande CA1312265 ou WO02052263, d'interposer au-dessus et au-dessous de la membrane poreuse hydrophile, une structure hydrophobe percée pour forcer le flux à passer par le spot de capture. Toutefois, avec ces dispositifs, il existe toujours un problème de diffusion centrifuge à partir du spot, et les éléments de révélation qui passent par le spot de capture et qui n'y sont pas fixés vont pouvoir diffuser de façon centrifuge dans la membrane poreuse hydrophile et être stockés en périphérie du spot. Les éléments de révélation vont alors échapper au rinçage lui aussi focalisé sur le spot. Il existe alors un phénomène de remonté de cet élément de révélation sous forme d'un retour par diffusion centripète vers le spot ce qui entraine une recoloration du spot même dans le cas de l'absence de l'analyte recherché. Ce phénomène empêche très rapidement de lire le test (généralement entre 5 et 15 minutes) car il y a apparition de résultats faussement positifs. Ceci est très problématique, car ces dispositifs ne permettent pas de réinterpréter ultérieurement le dispositif en cas de doute, d'erreur humaine ou de perte d'information.

Une autre problématique majeure des dispositifs d'immunofiltration existants actuellement est le contrôle de la vitesse de passage et dans certains cas du temps de préincubation. Ces temps sont importants car l'interaction entre l'élément de capture et l'analyte ainsi qu'entre l'analyte et l'élément de révélation possède une certaine cinétique. Cette cinétique décrit le nombre d'interactions réalisées en fonction du temps. Ainsi en fonction des couples agent de capture/analyte et agent de révélation/analyte, il faut pouvoir garantir un nombre suffisant d'interactions pour chacun des couples pour obtenir un signal suffisant. Dans le cas où l'interaction agent de capture/analyte est plus lente, il faut pouvoir réguler la vitesse du passage de l'échantillon à travers la membrane en conséquence. Dans le cas où c'est l'interaction entre l'élément de révélation et l'analyte qui est limitante, il est nécessaire de procéder à un temps de préincubation où l'élément de révélation et l'analyte sont en mélange au-dessus du spot de capture. Sans système particulier, le passage à travers une membrane hydrophile est rapide (500*µ*l/min).

Pour contrôler le flux, il a été proposé d'utiliser un piston, en particulier dans la demande US2008318342.

Pour contrôler le temps de préincubation, il a été proposé dans la demande WO03016902 d'utiliser un dispositif en deux parties : une partie supérieure comportant une zone de recueil d'échantillon et une membrane poreuse, et une partie inférieure comportant une membrane poreuse et une membrane absorbante. En position initiale ces deux blocs ne communiquent pas, le fluide ne peut pas traverser par capillarité. Après une manipulation mécanique, les deux zones sont en contact et le fluide peut s'écouler par capillarité. Il a également été proposé de déposer l'élément de capture sur une membrane hydrophobe et d'activer le passage par adjonction d'un tensio-actif.

Les approches mécaniques sont difficilement réalisables sous un robot car ils nécessitent le développement et l'utilisation de systèmes dédiés. Elles exposent aussi les professionnels à d'éventuelles projections lors de la manipulation.

L'adjonction d'un tensio-actif au moment de la réalisation du test pour amorcer le système est très délétère car il interfère fortement dans les interactions agents de capture / analytes. De plus, dans le cas spécifique de l'immunohématologie, les globules rouges utilisés comme agent de révélation ne sont pas compatibles avec les tensio-actifs sous forme liquide car la membrane des globules rouges est dissoute et elles se vident de leur hémoglobine.

Une autre méthode, décrite dans EP0334015, consiste à utiliser une membrane supplémentaire sous-jacente à la première pour contrôler le flux, mais le dispositif proposé ne permet pas de résoudre les problèmes liés à la diffusion de l'échantillon et de l'élément de révélation dans la membrane poreuse hydrophile. Les tests de diagnostic immuno-hématologiques existants présentent donc de nombreux inconvénients.

Une autre méthode encore décrite dans EP0408378, comprend l'utilisation d'une membrane hydrophobe dont les surfaces sont rendues entièrement hydrophiles. Cependant, cette zone réactionnelle hydrophile non délimitée spécifiquement ne permet pas d'utiliser des volumes plus faibles de réactifs déposés sur ladite zone réactionnelle.

Aussi, la présente invention vise à pallier les inconvénients de l'art antérieur en proposant un dispositif adapté pour le diagnostic immuno-hématologique in vitro par capillarité fiable, rapide, mobile et économique, simple de fabrication et d'utilisation, pouvant être miniaturisé et automatisé, et présentant une grande sensibilité.

Pour répondre à cet objectif, la présente invention propose un dispositif de diagnostic in vitro pour la détection, à partir d'un échantillon de sang ou d'un de ses composants, d'au moins une réaction entre un antigène de phénotype érythrocytaire et un anticorps dirigé spécifiquement contre cet antigène, caractérisé en ce qu'il comprend :
- un support, et
- une membrane poreuse hydrophobe d'épaisseur comprise entre 0,05mm et 1,5mm et dont le diamètre des pores est compris entre 2 et 30 *µ*m, ladite membrane comprenant au moins une zone réactionnelle hydrophile destinée à recevoir ledit échantillon, ladite zone réactionnelle ayant une surface inférieure à la surface de la membrane poreuse hydrophobe.

L'invention vise également l'utilisation de ce dispositif, en particulier des procédés de phénotypage de groupes sanguins érythrocytaires et de détection d'anticorps, mettant en oeuvre ce dispositif.

Avantageusement la présente invention permet de remédier à l'ensemble des inconvénients rencontrés dans les tests d'immunofiltration existants actuellement, en particulier en permettant :
- d'éviter les remontées d'agent de révélation responsables des faux positifs
- une sensibilité accrue en utilisant des volumes plus faibles
- une miniaturisation et une automatisation du système
- un contrôle de la cinétique des réactions sans nécessité de manipulation mécanique du dispositif.

D'autres caractéristiques et avantages ressortiront de la description qui va suivre de l'invention, description donnée à titre d'exemple uniquement, en regard des dessins annexés sur lesquels :
- la figure 1 représente un schéma d'un mode particulier de réalisation du dispositif selon l'invention vue en perspective,
- la figure 2A représente la membrane poreuse hydrophobe et la membrane absorbante du dispositif selon l'invention avec une première variante des zones réactionnelles hydrophiles,
- la figure 2B représente la membrane poreuse hydrophobe du dispositif selon l'invention avec une deuxième variante des zones réactionnelles hydrophiles,
- la figure 3A représente un schéma d'une coupe du dispositif selon l'invention avec une membrane poreuse hydrophobe correspondant à la variante représentée sur la figure 2A,
- la figure 3B représente un schéma d'une coupe du dispositif selon l'invention avec une membrane poreuse hydrophobe correspondant à la variante représentée sur la figure 2B,
- la figure 4A représente les résultats obtenus après utilisation du dispositif selon l'invention, sur une zone réactionnelle de la membrane poreuse hydrophobe représentée sur la figure 2A et la partie de la membrane absorbante sous-jacente, et
- la figure 4B représente les résultats obtenus après utilisation du dispositif selon l'invention, sur une zone réactionnelle de la membrane poreuse hydrophobe représentée sur la figure 2B.

Le dispositif 10 selon l'invention est un dispositif de diagnostic in vitro pour la détection, à partir d'un échantillon de sang ou d'un de ses composants, d'au moins une réaction entre un antigène de phénotype érythrocytaire et un anticorps dirigé spécifiquement contre cet antigène.

Il s'agit d'un dispositif pour le diagnostic in vitro par capillarité. Il est particulièrement adapté pour le diagnostic immuno-hématologique in vitro.

Par antigène de phénotype érythrocytaire ou antigène de groupe érythrocytaire ou antigène de groupe sanguin, on entend l'ensemble des molécules immunigènes présentes à la surface des globules rouges pouvant induire le cas échéant la production d'anticorps dirigés contre lui et/ou permettre la reconnaissance puis la destruction des globules rouges par le système immunitaire.

La réaction entre un antigène de phénotype érythrocytaire et un anticorps dirigé spécifiquement contre cet antigène est appelée réaction antigène-anticorps dans le reste de la description.

Par échantillon de sang ou d'un de ses composants, on entend le sang total ou l'un de ses composants choisis notamment parmi la fraction globule rouge, la fraction globule blanc, le plasma ou le sérum.

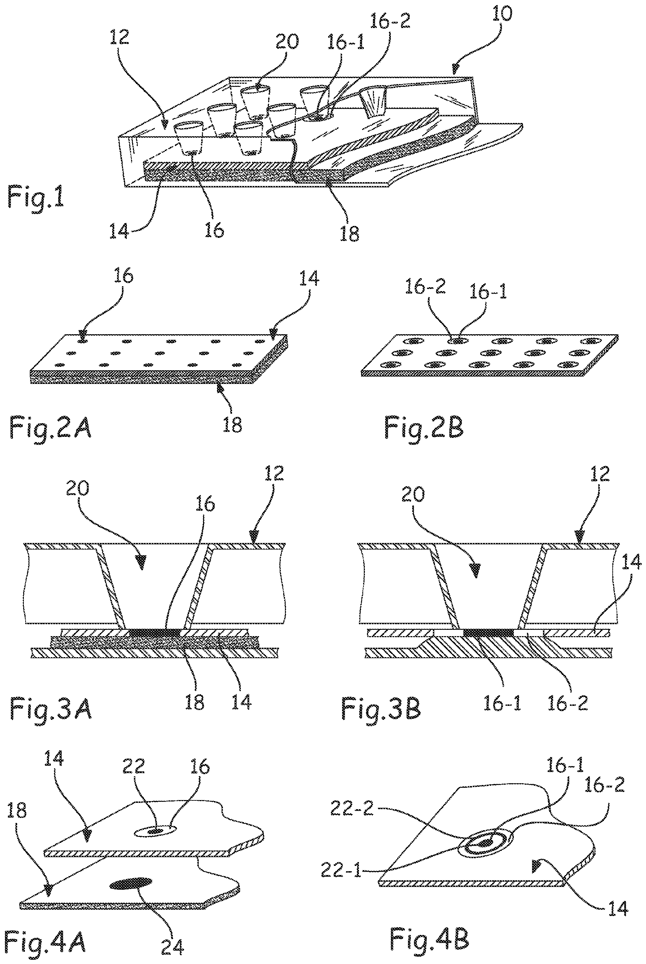

Comme représenté sur la figure 1, le dispositif 10 selon l'invention comprend :
- un support 12, et
- une membrane poreuse 14 hydrophobe comprenant au moins une zone réactionnelle 16 hydrophile destinée à recevoir l'échantillon à tester.

La membrane poreuse 14 a une épaisseur comprise entre 0,05mm et 1,5mm, préférentiellement entre 0,1 et 1mm, encore plus préférentiellement entre 0,4 et 0,8mm.

Le diamètre des pores est compris entre 2 et 30 *µ*m, préférentiellement entre 7 et 12*µ*m*.*

La membrane poreuse hydrophobe 14 peut être constituée de toute matière qui n'est pas altérée par les solvants aqueux. Cette matière peut notamment être choisie parmi des polymères naturels modifiés chimiquement ou non, comme par exemple le polymère de nitrocellulose, la cellulose ou parmi des polymères synthétiques comme par exemple le polyéthylène, polyéthylène haute densité (HDPE) ou les polymères fluorés tel que le PVDF. Cette matière doit être initialement hydrophobe où rendue hydrophobe par un traitement adéquat. Ces polymères peuvent être ou non fonctionnalisés avec des groupements réactifs capables de créer des liaisons avec les agents de capture utilisés ultérieurement.

La membrane poreuse hydrophobe 14 comprend au moins une zone réactionnelle 16 hydrophile. La zone réactionnelle 16 a une surface inférieure à la surface de la membrane poreuse hydrophobe 14, c'est-à-dire que la membrane 14 ne peut pas être entièrement hydrophilisée.

La ou les zones réactionnelle(s) hydrophile(s) 16 de la membrane poreuse 14 ont préférentiellement été rendue(s) hydrophile(s) par adjonction d'un détergent localement, sans modification des fonctions chimiques du substrat poreux par un traitement chimique ou physique préalable de la membrane poreuse hydrophobe 14.

Par détergent on entend tout agent hydrophilisant, c'est-à-dire toute substance capable de rendre hydrophile la membrane hydrophobe 14.

Le détergent utilisé peut être choisi parmi les détergents naturels, naturels modifiés chimiquement ou obtenus par synthèse chimique. Préférentiellement, il s'agit d'un tensio-actif non ionique, par exemple le Triton X-100, le Tween 20 ou la saponine.

Le détergent peut être dilué dans une solution aqueuse ou un solvant organique comme l'éthanol, à une concentration comprise entre 0,01 et 5%. Préférentiellement, le détergent utilisé pour rendre la membrane 14 hydrophile localement est utilisé à une dose comprise entre 0,01 et 2% (poids/volume).

La quantité de détergent utilisée, corrélée aux autres caractéristiques de la membrane (porosité et épaisseur en particulier) permet de contrôler la vitesse de passage des fluides qui traverse la membrane. Il est généralement admis qu'il ne faut pas dépasser une dose maximale de 0,1% pour le TritonX-100 et 0,05% pour le Tween pour rendre hydrophile une membrane destinée à recevoir un élément de capture. Or, du fait des caractéristiques particulières de la membrane 14 selon l'invention, les détergents capable de rendre cette membrane hydrophile localement peuvent être utilisés jusqu'à 2%, notamment pour le Triton X-100 ou le Tween-20, sans perturber la réactivité de la zone, ce qui facilite l'hydrophilisation de la membrane 14.

Une même membrane hydrophobe 14 peut comporter plusieurs zones réactionnelles hydrophiles 16, à conditions que ces zones ne se recoupent pas.

Les zones réactionnelles 16 peuvent se présenter sous toutes formes géométriques, mais préférentiellement sous forme de cercles ou spots de diamètre compris entre 0,3mm et 20mm.

La zone réactionnelle 16 peut être hydrophile sur toute l'épaisseur de la membrane poreuse 14 et/ou en surface.

La zone réactionnelle 16 peut présenter un seul degré d'hydrophilisation comme représenté sur les figures 2A, 3A et 4A. Cette configuration est particulièrement adaptée à la détection de la présence d'anticorps spécifique dans l'échantillon à analyser.

Selon une variante, la zone réactionnelle 16 peut comporter plusieurs zones présentant des degrés d'hydrophilisation différents.

Comme représenté sur la figure 2B, 3B et 4B, la zone réactionnelle 16 peut comporter deux zones hydrophiles 16-1, 16-2 avec un degré d'hydrophilisation plus important au centre 16-1 de la zone réactionnelle qu'en périphérie 16-2. Ces zones hydrophiles sont préférentiellement uniquement en surface de la membrane 14.

La zone réactionnelle 16 peut aussi comporter deux zones hydrophiles avec un degré d'hydrophilisation différent, l'une en surface, l'autre dans l'épaisseur.

Dans le cas où une zone réactionnelle 16 comporte deux zones hydrophiles, la zone réactionnelle 16 a été rendue hydrophile avec deux détergents différents sans modification des fonctions chimiques du substrat poreux par un traitement chimique ou physique préalable de la membrane poreuse.

Avantageusement, la configuration de la zone réactionnelle 16 avec deux régions présentant une hydrophilisation différente, en particulier en surface, telle que représentée sur les figures 2B, 3B et 4B, permet de répondre à une préoccupation particulière des professionnels de la transfusion, à savoir distinguer l'éventuelle coexistence de deux populations, l'une étant positive et l'autre négative, phénomène appelé double population. Cette configuration est particulièrement adaptée à la détection et l'identification d'antigènes particuliers dans l'échantillon à tester.

La zone réactionnelle hydrophile 16 de la membrane poreuse 14 peut comprendre des agents de capture. Les agents de capture sont absorbés ou liés de façon covalente à la membrane 14.

Par agents de capture on entend tout élément chimique ou biologique fixé sur la zone 16 capable de retenir l'analyte d'intérêt contenu dans l'échantillon à tester, seul ou complexé avec un agent de révélation.

Lorsque l'on cherche à identifier la présence d'anticorps particuliers dans l'échantillon à tester, ces agents de capture comprennent des antigènes de phénotype érythrocytaire particuliers. Il peut s'agir d'antigènes purifiés ou non, de fragments ou membranes de cellules portant les antigènes, des cellules vidées ou non portant les antigènes ou encore des protéines recombinantes ou des antigènes obtenus par synthèse. Préférentiellement, les agents de captures sont des hématies vidées d'hémoglobine, portant les antigènes.

Lorsque l'on cherche à identifier la présence d'antigènes particuliers dans l'échantillon à tester, les agents de capture sont des anticorps.

Les agents de capture, lorsqu'ils sont présents sur la zone réactionnelle 16, peuvent être déposés en même temps que le détergent servant à hydrophiliser la membrane 14 pour délimiter la zone réactionnelle 16, soit après hydrophilisation, indépendamment du détergent.

Les agents de capture peuvent être déposés sur la zone réactionnelle 16 dans un tampon non dénaturant constitué d'une solution de pH stabilisé entre pH4 et pH10, préférentiellement entre pH6,5 et pH7,8, encore plus préférentiellement entre pH 7 et pH 7,5. Les agents de capture peuvent soit être absorbés soit être liés de façon covalente.

Les agents de capture peuvent être additionnés d'adjuvants destinés à maintenir leur stabilité microbiologique tels que l'azide de sodium, des antibiotiques et sa stabilité conformationelle tels que des sucres (sucrose, dextrose, tréhalose), ainsi que tout autre agent connu de l'homme de l'art pour réaliser ces fonctions. Les agents de capture peuvent être présents sur toute la zone 16 ou sur une partie seulement.

Le détergent et/ou les agents de capture se présentent préférentiellement sous forme de solutions qui peuvent être déposées à l'aide de pipeteurs robotisés ou manuels. Avantageusement, ces solutions peuvent également facilement être déposées en utilisant des aiguilles qui retiennent par capillarité le volume désiré.

Ces aiguilles peuvent être en toutes matières, mais plus particulièrement métalliques, et présenter ou non un revêtement hydrophobe. Leurs extrémités peuvent être plates ou présenter une encoche d'un volume déterminé.

Le dépôt des solutions de détergent et/ou d'agents de capture, doit être suivi d'un séchage de la membrane, la durée du séchage dépendant de la température appliquée : au moins 4 heures à température ambiante, au moins une heure à 37°C.

Le dispositif selon l'invention, peut comprendre une membrane absorbante 18 sous la membrane poreuse 14. Cette membrane 18 permet d'absorber les liquides déposés au niveau des zones réactionnelles 16, qui ne sont pas retenus par la membrane 14, en particulier lorsque la zone réactionnelle 16 est hydrophile sur toute l'épaisseur de la membrane 14.

La membrane 18 peut être constituée en une matière permettant une absorption passive par capillarité comme du papier absorbant, de la cellulose, etc., ou en polymères absorbants. A titre d'exemple, on peut citer les produits suivants :
- Millipore C048, C068, C083, C248
- Whatman CF3, CF4, CF10, Grade 470, CF5, CF6, CF7, Grade 900, Grade 300
- Ahlstrom Grades 601, 642, 631, 238, 237, 222, 243, 320
- Pall Grades 111, 113, 133, 165, 197, 8975, 8964, 8301, Accuwik® Ultra
- Cleanis Gelmax superabsorbant pad
- Coton

La composition de la membrane absorbante 18 et ses dimensions doivent être choisies de façon à pouvoir absorber l'ensemble des solutions utilisées lors du test (Vₜₒₜₐₗ en *µ*l). Chaque membrane étant caractérisée par une capacité d'absorption (C en *µ*l/cm²), on choisit la membrane et ses dimensions (D en cm²) pour satisfaire à l'équation suivante : D > Vₜₒₜₐₗ/*C*.

De manière alternative, le liquide peut être absorbé par un différentiel de pression entre la zone au-dessus de la membrane et la zone en dessous de celle-ci. Par exemple en utilisant un système d'aspiration à vide partiel.

La membrane 14 et éventuellement la membrane 18, sont disposées dans un support 12.

Le support 12 du dispositif 10 selon l'invention est préférentiellement un support rigide. Il peut s'agir par exemple d'une coque.

De façon préférée, le support est constitué en matériau rigide ne pouvant pas laisser échapper de liquide. Il peut s'agir en particulier de matières plastiques, telles que le polypropylène, le polyéthylène, le polystyrène, l'acrylonitrile butadiène styrène, polyéthylène téréphtalate, polycarbonate, polyamide, chlorure de polyvinyle, poly-méthacrylate de méthyle.

Le support 12 comporte préférentiellement au moins une lumière 20, chaque lumière 20 venant au droit de chaque zone réactionnelle hydrophile 16 de la membrane poreuse 14. Cette lumière 20 correspond à la zone de recueil de l'échantillon déposé sur la zone réactionnelle hydrophile 16. La zone hydrophile 16 peut être de taille identique à celle du fond de la lumière, plus petite ou plus grande, sous réserve que deux zones hydrophiles soient toujours séparées par une zone hydrophobe sur la membrane 14.

Les lumières 20 peuvent avoir des bords transparents permettant le cas échéant de visualiser les signaux sous-jacents.

La zone de recueil doit être d'une dimension telle qu'elle puisse contenir au moins le volume maximum d'échantillon à tester déposé sur la membrane réactionnelle 16.

L'indépendance de chaque zone réactionnelle 16 est obtenue par la barrière que constitue la membrane hydrophobe entre les zones réactionnelles. Cette indépendance permet de réaliser plusieurs diagnostics différents sur un même dispositif comportant une seule membrane. Selon une variante, cette indépendance peut aussi éventuellement être matérialisée en segmentant le support 12 en unités physiquement indépendantes séparées par des cloisons contenant chacune sa propre membrane.

Par ailleurs, il est possible d'améliorer le contact entre les membranes 14 et 18 en réalisant autour de la lumière 20 une surépaisseur, et au fond de la lumière un dôme en regard de l'ouverture de ladite lumière 20.

Le support 12 peut être une coque présentant plusieurs lumières 20, de dimensions compatibles avec le standard SBS/AINSI dans ses dimensions extérieures.

Le dispositif 10 selon l'invention peut être utilisé dans la détermination de la présence ou l'absence d'un analyte dans un fluide biologique, en particulier dans le sang ou l'un de ses constituants par capillarité. Cet analyte peut être un antigène de phénotype érythrocytaire ou un anticorps dirigé contre cet antigène.

Le dispositif 10 peut être utilisé notamment pour :
- Le phénotypage de globules rouges, c'est à dire la détermination des antigènes à leur surface.
- L'épreuve dite de Simmonin qui identifie la présence d'anticorps anti-A ou anti-B.
- La recherche ou l'identification d'anticorps dirigés contre des antigènes cellulaires en particulier érythrocytaire dans le cadre de la recherche d'allo-anticorps, d'auto-anticorps ou encore d'agglutinines froides.

De façon spécifique l'invention vise donc l'utilisation d'un dispositif 10 pour identifier et déterminer des groupes sanguins ABO, le phénotypage Rhésus étendu, la recherche d'agglutinine irrégulière, la recherche d'auto-anticorps, la recherche d'antiglobuline froide et/ou la validation croisée, partir d'un échantillon de sang ou d'un de ses composants.

L'utilisation du dispositif 10 selon l'invention nécessite l'utilisation d'un agent de révélation. Préférentiellement il s'agit d'hématies. Ces hématies :
- sont des hématies de phénotype connu, appelées hématies tests, pour l'utilisation du dispositif pour la recherche d'anticorps ou l'épreuve de Simmonin,
- sont celles contenues dans l'échantillon à tester pour l'utilisation du dispositif pour le phénotypage.

En fonction de l'analyte dont on cherche à détecter la présence dans l'échantillon à tester, il faut faire varier la nature de l'agent de capture, la nature de l'agent de révélation, la méthode d'hydrophilisation (simple ou multiple, en épaisseur ou en surface) et le protocole suivi.

Dans tous les cas, avant dépôt de l'échantillon à tester sur la zone réactive, il est possible de procéder à une hydratation de la zone réactionnelle 16 à l'aide d'une solution tampon. Ce tampon peut être constitué d'une solution de pH stabilisé entre pH6 et pH8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (Tween 20 0,01 à 0,05% m/v), des agents de saturation (BSA) et/ou des agents capables de potentialiser les réactions antigène-anticorps.

De même, pour pouvoir lire les résultats il est nécessaire d'utiliser un tampon de rinçage. Ce tampon de lavage est préférentiellement constitué de PBS, TBS ou solution saline de pH compris entre 2 et 10, préférentiellement entre 5 et 9. L'osmolarité du tampon doit être contrôlée pour éviter l'hémolyse des globules rouges. Le tampon doit être choisi pour ne pas détacher les agents de révélation ou les analytes colorés fixés directement ou indirectement aux agents de capture. De façon surprenante, pour l'utilisation du dispositif selon l'invention il est préférable d'utiliser des solutions de lavage légèrement hyperosmotiques (c'est-à-dire entre 300m*O*sm et 800 m*O*sm) obtenues par la présence d'agents salins comme le NaCl ou d'osmolites non ioniques comme par exemple la glycine ou la taurine. Ce tampon peut être coloré avec des couleurs contrastantes avec la couleur de l'agent de révélation. Par exemple si les agents de révélation sont des hématies, la solution tampon de lavage peut être colorée en bleu ou vert. Il est également possible d'ajouter une faible dose de tensio-actif dans la solution de lavage pour éliminer le bruit de fond. Ces tensio-actifs sont préférentiellement des tensio-actifs non ioniques en particulier les esters de sucre, notamment les esters de sorbitan polyoxyéthyléniques (Tween).

Lors de l'utilisation du dispositif 10, les dépôts de liquides peuvent se faire notamment à l'aide d'un système pipeteur ou à l'aide d'un système de réplication par capillarité.

Avantageusement, le dispositif selon l'invention ne nécessite ni centrifugation, ni agitation, ni mise sous vide, ni dispositif ad hoc. Il peut aussi bien être utilisé manuellement de façon totalement autonome, qu'être facilement automatisé sur des robots. Les différentes caractéristiques de la membrane 14 permettent de contrôler la vitesse de passage et d'obtenir des temps de passages suffisamment longs pour permettre la réaction antigène-anticorps, malgré des tailles de porosité de la membrane 14 pourtant importantes.

Selon une première variante, l'invention vise l'utilisation du dispositif 10 pour le phénotypage des globules rouges.

L'objectif est de rechercher des antigènes à la surface de globules rouges. Dans ce cas, on utilise comme agent de capture un anticorps ou un mélange d'anticorps, capable de reconnaitre spécifiquement l'antigène en question ou un variant d'un antigène. L'anticorps peut être un anticorps monoclonal purifié ou semi purifié, un surnageant de culture contenant l'anticorps monoclonal (voir Tableau 1) ou un anticorps polyclonal, un anti-sérums. On peut aussi utiliser des agglutinines ou des lectines.

De façon non exhaustive, on peut citer les anticorps monoclonaux suivant :

**Tableau 1 : liste non exhaustive des anticorps de captures utilisables pour le phénotypage globulaire**

| Antigène recherché | Elément de capture | Ex. référence clones | Elément de révélation |
|---|---|---|---|
| A | Ac Anti-A | BIRMA-1, | Globules rouges de l'échantillon |
| B | Ac Anti-B | LB-2 et/ou ES-4 | Globules rouges de l'échantillon |
| AB | Ac Anti-AB | ES-4 et/ou ES-15 et/ou BH517 | Globules rouges de l'échantillon |
| D (RH1) | Ac Anti-D | RUM-1 et/ou MS-201 et/ou MAD-2 et/ou TH-28 et/ou MS-26 | Globules rouges de l'échantillon |
| C (RH2) | Ac Anti-C | MS-273 ou MS-24 | Globules rouges de l'échantillon |
| c (RH4) | Ac Anti-c | MS-33 | Globules rouges de l'échantillon |
| E (RH3) | Ac Anti-E | MS-258, MS-80 | Globules rouges de l'échantillon |
| e (RH5) | Ac Anti-e | MS-16, MS-21, MS-63 | Globules rouges de l'échantillon |
| K | Ac Anti-K | MS-56 | Globules rouges de l'échantillon |
| Fya (FY1) | Ac Anti-Fya | P3TIM | Globules rouges de l'échantillon |
| Fyb (FY2) | Ac Anti-Fyb | | Globules rouges de l'échantillon |
| Jka (JK1) | Ac Anti-Jka | MS-15 | Globules rouges de l'échantillon |
| Jkb (JK2) | Ac Anti-Jkb | MS-8 | Globules rouges de l'échantillon |
| S (MNS3) | Ac Anti-S | MS-94 | Globules rouges de l'échantillon |
| s (MNS4) | Ac Anti-s | P3BER L | Globules rouges de l'échantillon |
| Lea (LE1) | Ac Anti-Lea | LM112/161 | Globules rouges de l'échantillon |
| Leb (LE2) | Ac Anti-Leb | LM129/181 | Globules rouges de l'échantillon |
| M (MNS1) | Ac Anti-M | M110/140 | Globules rouges de l'échantillon |
| N (MN52) | Ac Anti-N | | Globules rouges de l'échantillon |
| P1 | Ac Anti-Pl | P3MON23 | Globules rouges de l'échantillon |
| Kpa (KEL3) | Ac Anti-Kpa | | Globules rouges de l'échantillon |
| Lua (LU1) | Ac Anti-Lua | | Globules rouges de l'échantillon |
| Lub (LU2) | Ac Anti-Lub | | Globules rouges de l'échantillon |
| k, cellano(KEL 2) | Ac Anti-k | | Globules rouges de l'échantillon |
| Kpb (KEL4) | Ac Anti-Kpb | | Globules rouges de l'échantillon |
| Cw | Ac Anti-Cw | | Globules rouges de l'échantillon |

Selon un mode de réalisation particulier, l'invention vise un procédé de phénotypage de groupes sanguins érythrocytaires, à partir d'un échantillon de globules rouges (globules rouges purs ou sang total), permettant la détection simultanée d'une population de phénotype positif et d'une population de phénotype négatif (double population), comprenant les étapes suivantes :
- déposer une solution pour hydrater la membrane poreuse 14 au centre de la zone réactionnelle d'un dispositif 10 selon l'invention, ladite zone réactionnelle 16 comportant deux zones hydrophiles 16-1, 16-2 avec un degré d'hydrophilisation plus important au centre 16-1 de la zone réactionnelle qu'en périphérie 16-2, et comportant au centre des agents de capture comprenant des anticorps ; la solution pour l'hydratation de la membrane est une solution connue pour être propice aux réactions immuno-hématologiques contenant éventuellement des additifs, comme par exemple un tampon constitué d'une solution de pH stabilisé entre pH6 et pH 8,5, préférentiellement entre pH 6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05%m/v), des agents de saturation (par exemple BSA) et/ou des agents capables de potentialiser les réactions antigène-anticorps. Ce tampon peut éventuellement présenter une activité protéasique par exemple obtenue par l'ajout d'enzymes telles que la papaïne ou la bromelain. Ce tampon peut éventuellement contenir des agents polycationiques comme le polybrene ou la polylysine, permettant de potentialiser la réaction et de maintenir plus longtemps le globules en contact avec l'élément de capture ;
- diluer les globules rouges à phénotyper dans une solution tampon, à savoir une solution tampon connue pour être propice aux réactions immuno-hématologiques contenant éventuellement des additifs comme par exemple un tampon constitué d'une solution de pH stabilisé entre pH6 et pH8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05%m/v), des agents de saturation (par exemple BSA) et/ou des agents capables de potentialiser les réactions antigène-anticorps. Ce tampon peut éventuellement présenter une activité protéasique par exemple obtenue par l'ajout d'enzymes telles que la papaïne ou la bromelain. Ce tampon peut éventuellement contenir des agents polycationiques comme le polybrene ou la polylysine, permettant de potentialiser la réaction et de maintenir plus longtemps les globules en contact avec les éléments de capture ;
   - ajouter cette solution contenant les globules rouges à phénotyper au centre de la zone réactionnelle 16-1 ;
   - incuber, préférentiellement à une température comprise entre 15 et 40°C, en particulier entre 18°C et 25°C, pendant une durée de 60 secondes à 15min en particulier pendant 2min à 10 min ;
   - déposer une solution de rinçage sur la zone réactionnelle ; la solution de rinçage étant une solution connue pour ne pas être délétère pour les réactions antigènes-anticorps, maintenant l'intégrité des globules rouges et capable de défaire les intéractions non spécifiques, comme par exemple un tampon constitué d'une solution de pH stabilisé entre pH6 et pH8,5, préférentiellement entre pH 6,5 et pH7,8, en particulier entre pH7 à pH7,5 et d'osmolarité comprise entre 250m*O*sm et 800m*O*sm, préférentiellement entre 300m*O*sm et 600m*O*sm.

Cette solution peut éventuellement contenir des détergents en faible concentration (notamment Tween 20 de 0,01 à 0,05% m/v), des agents de saturation (par exemple BSA) et/ou des agents capables de potentialiser les réactions antigène-anticorps. Dans le cas d'utilisation préalable d'agent polycationique, ce tampon devra être fortement ionique, par exemple, elle devra contenir plus de 100mM de NaCl pour pouvoir défaire les interactions non spécifiques provoquées par ce type d'additif.

L'échantillon à déposer peut être des globules rouges ou du sang total. L'échantillon peut être dilué dans un tampon constitué d'une solution de pH stabilisé entre pH6 et pH8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05% m/v), des agents de saturation (par exemple BSA) et/ou des agents capables de potentialiser les réactions antigène-anticorps. Ce tampon peut éventuellement présenter une activité protéasique par exemple obtenue par l'ajout d'enzymes telles que la papaïne ou la bromelain.

L'hydrophilisation des zones réactionnelles 16 de la membrane 14 du dispositif 10 utilisé pour la mise en oeuvre de ce procédé est préférentiellement une hydrophilisation en surface.

L'hydrophilisation peut par exemple être réalisée à l'aide d'une solution de Tween 20 dans l'éthanol de concentration comprise entre 0,1%m/v et 1%m/v, préférentiellement 0,2%m/v et 1%m/v de volume compris entre 2*µ*l et 40*µ*l et préférentiellement entre 5*µ*l et 20*µ*l au centre de laquelle est créée une zone d'hydrophilisation dans l'épaisseur réalisée à l'aide d'une solution aqueuse de Triton X100 de concentration comprise entre 0,1%m/v et 2%m/v, préférentiellement 0,5%m/v et 1%m/v de volume compris entre 25nl et 15*µ*l et préférentiellement entre 100nl et 10*µ*l.

Si les globules rouges présents dans l'échantillon portent l'antigène reconnu par l'élément de capture, les globules rouges restent immobilisés au centre de la zone réactionnelle 16 de la membrane 14 malgré le rinçage et la zone réactionnelle centrale 16-1 reste rouge. Si les globules rouges présents dans l'échantillon ne portent pas l'antigène reconnu par l'élément de capture, les globules rouges sont chassés par le rinçage : la zone centrale 16-1 reste blanche et il se forme un anneau rouge dans la zone périphérique 16-2. Si l'échantillon contient deux populations différentes, on observe à la fois un centre rouge 22-1 et un anneau périphérique rouge 22-2.

La lecture des résultats peut être visuelle ou automatique.

Selon un autre mode de réalisation particulier, l'invention vise un procédé de phénotypage de groupes sanguins érythrocytaires, à partir d'un échantillon de globules rouges (globules rouges purs ou sang total), ne permettant pas la détection de doubles populations, comprenant les étapes suivantes :
- déposer une solution pour hydrater la membrane poreuse 14 au centre de la zone réactionnelle d'un dispositif 10 selon l'invention, ladite zone réactionnelle 16 comportant une seule zone hydrophile et comportant des agents de captures comprenant des anticorps ; la solution pour l'hydratation de la membrane est une solution connue pour être propice aux réactions immuno-hématologiques contenant éventuellement des additifs, comme par exemple un tampon constitué d'une solution de pH stabilisé entre pH6 et pH8,5, préférentiellement entre pH 6,5 et pH 7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05%m/v), des agents de saturation (par exemple BSA) et/ou des agents capables de potentialiser les réactions antigène-anticorps. Ce tampon peut éventuellement présenter une activité protéasique par exemple obtenue par l'ajout d'enzymes telles que la papaïne ou la bromelain. Ce tampon peut éventuellement contenir des agents polycationiques comme le polybrene ou la polylysine, permettant de potentialiser la réaction et de maintenir plus longtemps les globules rouges en contact avec les éléments de capture ;
- éventuellement diluer les globules rouges à phénotyper dans une solution tampon, à savoir une solution tampon connue pour être propice aux réactions immuno-hématologiques contenant éventuellement des additifs, comme par exemple un tampon constitué d'une solution de pH stabilisé entre pH6 et pH8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05%m/v), des agents de saturation (par exemple BSA) et/ou des agents capables de potentialiser les réactions antigène-anticorps. Ce tampon peut éventuellement présenter une activité protéasique par exemple obtenue par l'ajout d'enzymes telles que la papaïne ou la bromelain. Ce tampon peut éventuellement contenir des agents polycationiques comme le polybrene ou la polylysine, permettant de potentialiser la réaction et de maintenir plus longtemps le globules en contact avec l'élément de capture ;

- ajouter cette solution contenant les globules rouges à phénotyper au centre de la zone réactionnelle,
- incuber, préférentiellement à une température comprise entre 15 et 40°C en particulier entre 18°C et 25°C, pendant une durée de 2 secondes à 15min et en particulier pendant 1min à 10 min.
- déposer une solution de rinçage sur la zone réactionnelle la solution de rinçage étant une solution connue pour ne pas être délétère pour les réactions antigènes-anticorps, maintenant l'intégrité des globules rouges et capable de défaire les interactions non spécifiques, comme par exemple un tampon constitué d'une solution de pH stabilisé entre pH6 et pH 8,5, préférentiellement entre pH6,5 et pH7,8, préférentiellement entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05%m/v), des agents de saturation (par exemple BSA) et/ou des agents capables de potentialiser les réactions antigène-anticorps. Dans le cas d'utilisation préalable d'agent polycationique, ce tampon devra être fortement ionique, par exemple, elle devra contenir plus de 100mM de NaCl pour pouvoir défaire les interactions non spécifiques provoquées par ce type d'additif.

L'hydrophilisation des zones réactionnelles 16 de la membrane 14 du dispositif 10 utilisé pour la mise en oeuvre de ce procédé peut être une hydrophilisation dans l'épaisseur. Il est nécessaire que le dispositif 10 comprenne également un système d'absorption sous-jacent à la membrane 14, par exemple une membrane absorbante 18.

L'hydrophilisation peut être réalisée à l'aide d'une solution aqueuse de Triton X100 de concentration comprise entre 0,1%m/v et 2%m/v, préférentiellement 0,5%m/v et 1%w/v de volume compris entre 25nl et 15*µ*l et préférentiellement entre 100nl et 10*µ*l.

Si les globules rouges présents dans l'échantillon portent l'antigène reconnu par l'élément de capture, les globules rouges restent immobilisés au centre 22 de la zone réactionnelle 16 de la membrane 14 malgré le rinçage et la zone réactionnelle 16 reste rouge. Si les globules rouges présents dans l'échantillon ne portent pas l'antigène reconnu par l'élément de capture, les globules rouges sont chassés par le rinçage et la zone réactionnelle 16 reste blanche. La membrane absorbante 18 absorbe (zone 24) tout ce qui ne s'est pas fixé sur la membrane 14.

La lecture des résultats peut être visuelle ou automatique.

Selon une deuxième variante, l'invention vise l'utilisation du dispositif 10 pour la détection d'anticorps multivalents dans le sang ou épreuve de Simonin.

L'objectif est de rechercher des anticorps spécifiques. L'élément de capture comprend donc l'antigène contre lequel l'anticorps recherché est dirigé.

Les antigènes immobilisés peuvent être des antigènes synthétiques couplés ou non à un polymère ou à une structure protéique. Il peut s'agir aussi de protéines recombinantes contenant une ou plusieurs séquences de l'antigène ou d'un mélange d'antigène de variants souhaités. Les antigènes immobilisés peuvent aussi être sur des cellules ou des fragments de cellules. En particulier des fragments membranaires ou des cellules vidées de leur contenu cytoplasmique.

Ces cellules peuvent être en particulier des globules rouges vidée de leur cytoplasme que l'on appelle également « ghosts ».

Si l'échantillon possède des anticorps dirigés contre l'antigène en question, ceux-ci resteront capturés à la surface de la membrane 14 au niveau de la zone réactionnelle 16.

Il est nécessaire d'utiliser un élément de révélation pour révéler la présence de ces anticorps soit en reconnaissant leur nature d'anticorps (anti-IgG, anti-IgM), soit si l'anticorps mis en jeu est multivalent (capable de détecter plusieurs antigènes simultanément) on peut utiliser un élément de révélation portant aussi l'antigène en question.

**Tableau 2 : Exemples concernant la recherche d'anticorps dans le domaine de l'immunohématologie**

| Antigène recherché | Elément de capture | Elément de révélation |
|---|---|---|
| IgM Anti-A | Ghosts A | Hématies test A1 |
| IgM Anti-B | Ghosts B | Hématies test B |

L'invention vise donc également un procédé de détection des anticorps multivalents présents dans le sang, à partir d'un échantillon de plasma, de sérum ou de sang total, comprenant les étapes suivantes :
- déposer l'échantillon à tester sur la zone réactionnelle 16 d'un dispositif 10, ladite zone réactionnelle 16 comportant des agents de captures comprenant des antigènes,
- ajouter des hématies tests de phénotype connu, comportant les mêmes antigènes que les agents de capture,
- laisser le mélange passer à travers la zone hydrophile, ladite zone hydrophile présentant des caractéristiques permettant un temps de passage compris entre 1 et 45 minutes, préférentiellement entre 3 et 15 minutes,
- déposer une solution de rinçage sur la zone réactionnelle, comme par exemple un tampon constitué d'une solution de pH stabilisé entre pH6 et pH8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (notamment Tween 20 de 0,01 à 0,05%m/v), des agents de saturation (par exemple BSA).

L'échantillon à déposer peut être du plasma, du sérum ou du sang total dilué ou non dans un tampon constitué d'une solution de pH stabilisé entre pH6 et pH8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (par exemple Tween 20 0,01 à 0,05%m/v), des agents de saturation (notamment BSA) et/ou des agents capables de potentialiser les réactions antigène-anticorps. Ce tampon est préférentiellement de salinité inférieure à 100mM en NaCl.

L'hydrophilisation des zones réactionnelles 16 de la membrane 14 du dispositif 10 utilisé pour la mise en oeuvre de ce procédé est préférentiellement réalisée en épaisseur. Il est donc nécessaire que le dispositif 10 comprenne également un système d'absorption sous-jacent à la membrane 14, par exemple une membrane absorbante 18.

L'hydrophilisation peut être réalisée à l'aide d'une solution aqueuse de Triton X100 de concentration comprise entre 0,3%m/v et 2%m/v, préférentiellement 0,5%m/v et 1%w/v de volume compris entre 25nl et 15*µ*l et préférentiellement entre 100nl et 10*µ*l. Les agents de capture peuvent être déposés sur la membrane, mélangés au détergent, ou après hydrophilisation des zones réactionnelles 16.

Si le plasma testé contient des anticorps dirigés contre l'antigène présent dans l'agent de capture et dans l'agent de révélation (hématies tests), un centre rouge 22 apparaît dans la zone réactionnelle 16. Un centre blanc signale l'absence dans le plasma testé des anticorps dirigés contre l'antigène présent à la fois dans l'agent de capture et dans l'agent de révélation (hématies tests). La membrane absorbante 18 absorbe (zone 24) tout ce qui ne s'est pas fixé sur la membrane 14.

La lecture des résultats peut être visuelle ou automatique.

Selon une troisième variante, l'invention vise l'utilisation du dispositif 10 pour la recherche d'agglutine irrégulière dans le sang.

L'objectif est d'identifier la présence d'hématies sensibilisées par des anticorps plasmatiques de l'échantillon ou par une activation du complément subséquente. Dans ce format, soit il n'y a pas d'agent de capture, soit celui-ci est un agent d'agrégation comme un polymère polycationique.

Après avoir incubé des hématies test de panel avec le plasma, on ajoute au mélange un agent capable d'agréger de façon réversible les globules rouges. Cet agent d'agrégation réversible peut être choisi parmi les polymères polycationiques comme par exemple le polybrène, la polylysine ou la polyéthyleneinmine. Les hématies restent alors retenues et forment un bouton de globules car la taille des agrégats formés est trop importante pour leur permettre de traverser la membrane. On rince les globules de l'excès de globulines non spécifiques à l'aide d'une solution contenant aussi un agent d'agrégation afin d'éviter la déstabilisation du bouton. Après avoir rincé ces globules retenus, on ajoute un réactif de Coombs multivalent permettant de réaliser une réticulation des cellules du bouton si celles-ci sont sensibilisées.

L'ajout d'un tampon salin et contenant une faible dose de tensio-actif permet de rompre les agrégats de cellules non sensibilisées et de garder les agrégats de cellules sensibilisées réticulées.

**Tableau 3 : liste non exhaustive des anticorps utilisables dans la recherche d'agglutinines irrégulières**

| Analyte recherché | Agent de réticulation | Référence clone | |
|---|---|---|---|
| Ig*G* humain sur hématie test | Anti-Ig*G*, protéine *A*, protéine *G*.. | MS-278, Protéine *A*, *G*, *A*/*G* | hématie test |
| IgM humain sur hématie test | Anti-IgM | | hématie test |
| *C*3d humain sur hématie test | Anti-*C*3d | BRIC-8 | hématie test |

Pour la mise en oeuvre de ces procédés, l'hydrophilisation des zones réactionnelles 16 de la membrane 14 du dispositif 10 utilisé pour la mise en oeuvre de ce procédé est préférentiellement réalisée en épaisseur. Il est donc nécessaire que le dispositif 10 comprenne également un système d'absorption sous-jacent à la membrane 14, par exemple une membrane absorbante 18.

L'hydrophilisation peut être réalisée à l'aide d'une solution aqueuse de Triton X100 de concentration comprise entre 0,3%m/v et 2%m/v, préférentiellement 0,5%m/v et 1%w/v de volume compris entre 25nl et 15*µ*l et préférentiellement entre 100nl et 10*µ*l. On peut avantageusement adjoindre un agent d'aggrégation réversible, préférentiellement choisi parmi les polymères polycationiques comme par exemple le polybrene, la polylysine ou la polyethyleneinmine.

Selon cette troisième variante, l'invention vise donc également un procédé de détection des anticorps anti-érythrocytaires présents dans le sang, de validation croisée ou de recherche d'auto-anticorps ou d'agglutinine froide, à partir d'un échantillon de plasma, de sérum ou de sang total, caractérisé en ce qu'il comprend les étapes suivantes :
- pour la recherche d'alloanticorps, incuber préalablement l'échantillon à tester avec un tampon, des hématies tests de phénotype connu et un agent capable d'agréger les globules rouges à une température comprise entre 15 et 40°C préférentiellement à 37°C environ, pendant une durée comprise entre 3 et 60 minutes, préférentiellement entre 5 et 30 minutes ; le tampon est un tampon de basse force ionique, tel qu'un tampon LISS (par exemple contenant moins de 50 mM de NaCl) ; pour la recherche d'autoanticorps, utiliser simplement l'échantillon contenant les hématies de l'échantillon sans incubation préalable ;
- ajouter à ce mélange un agent capable d'agréger les globules, par exemple une solution d'hexadimethrine bromide ;
- après une période préférentiellement comprise entre 15 secondes et 5 minutes, déposer le mélange sur la zone réactionnelle 16 d'un dispositif 10 selon l'invention, et laisser s'écouler ; il se forme un bouton de cellules au-dessus du spot,
- déposer une solution contenant l'agent capable d'agréger les globules rouges sur la zone réactionnelle, l'agent capable d'agréger les globules rouges peut être par exemple une solution d'hexadimethrine bromide, préférentiellement avec une concentration en hexadimethrine bromide dans la solution comprise entre 0,01 et 2% (m/v), encore plus préférentiellement entre 0,05 et 0,5% ; cet agent permet de réaliser un rinçage des protéines non spécifiques tout en maintenant l'intégrité du bouton de cellules ;
- déposer un réactif de Coombs, d'antiglobuline humaine ou d'anti-complément sur la zone réactionnelle, et
- déposer une solution de rinçage sur la zone réactionnelle comme par exemple une solution saline hypertonique de pH stabilisé entre pH 6 et pH 8,5, préférentiellement entre pH 6,5 et pH 7,8, préférentiellement entre pH 7 à pH 7,5 et d'osmolarité comprise entre 300m*O*sm et 800m*O*sm. Cette solution peut éventuellement contenir des détergents en faible concentration (par exemple Tween 20 0,01 à 0,05%m/v) et un colorant d'une couleur contrastant avec le rouge (bleu ou vert).

L'échantillon à déposer peut être du plasma, du sérum ou du sang total dilué ou non dans un tampon constitué d'une solution de pH stabilisé entre pH 6 et pH 8,5, préférentiellement entre pH6,5 et pH7,8, en particulier entre pH7 à pH7,5 et d'osmolarité comprise entre 250m*O*sm et 800m*O*sm, préférentiellement entre 300m*O*sm et 600m*O*sm. Cette solution peut éventuellement contenir des détergents en faible concentration (par exemple Tween 20 0,01 à 0,05%m/v), des agents de saturation (notamment BSA) et/ou des agents capables de potentialiser les réactions antigène-anticorps. Ce tampon est préférentiellement de salinité inférieure à 50mM en NaCl.

Si le plasma testé contient des anticorps dirigés contre l'antigène présent dans l'agent de révélation (hématies tests) ou si les hématies de l'échantillon sont déjà sensibilisées in vivo, un centre rouge 22 apparaît dans la zone réactionnelle 16.

Un centre blanc signale l'absence ou une dose non détectable d'anticorps dirigés contre l'antigène présent dans l'agent de révélation (hématies tests). La membrane absorbante 18 absorbe (zone 24) tout ce qui ne s'est pas fixé sur la membrane 14.

La lecture des résultats peut être visuelle ou automatique.

Le dispositif 10 selon l'invention peut se décliner suivant une gamme regroupant sur une même carte les tests complémentaires pertinents effectués le plus souvent simultanément par les professionnels, l'ensemble des tests étant effectués et interprétés de façon similaire.

Sur chacune des cartes, on va pouvoir détecter plusieurs analytes disposés en colonne pour plusieurs échantillons (donneurs ou patients) disposés en ligne. Il peut s'agir par exemple d'une carte :
- de groupage sanguin ABO-D,
- de groupage Rhesus-Kell,
- de recherche d'agglutinine irrégulière (3 phénotypes erythrocytaires),
- d'identification des agglutinines irrégulières (10 phénotypes érythrocytaires),
- de phénotypage étendu,
- de contrôle directe à l'antiglobuline,
- d'épreuve de compatibilité directe.

Le dispositif 10 selon l'invention peut être présenté dans un kit comprenant également les réactifs nécessaires à la réalisation d'au moins un des procédés d'utilisation dudit dispositif.

## Revendications

1. Dispositif (10) de diagnostic in vitro, pour la détection d'au moins une réaction entre un antigène de phénotype érythrocytaire et un anticorps dirigé spécifiquement contre cet antigène, à partir d'un échantillon de sang ou d'un de ses composants, **caractérisé en ce qu'**il comprend :
- un support (12), et
- une membrane poreuse hydrophobe (14) d'épaisseur comprise entre 0,05mm et 1,5mm et dont le diamètre des pores est compris entre 2 et 30 µm, ladite membrane comprenant au moins une zone réactionnelle hydrophile (16) destinée à recevoir ledit échantillon, la surface de la zone réactionnelle hydrophile (16) étant inférieure à la surface de la membrane poreuse hydrophobe (14).

2. Dispositif (10) de diagnostic selon la revendication 1, **caractérisé en ce que** la zone réactionnelle hydrophile (16) de la membrane poreuse (14) a été rendue hydrophile avec un détergent sans modification des fonctions chimiques du substrat poreux par un traitement chimique ou physique préalable de la membrane poreuse.

3. Dispositif (10) de diagnostic selon la revendication 2, **caractérisé en ce que** le détergent est un tensio-actif non ionique.

4. Dispositif (10) de diagnostic selon l'une des précédentes revendications, **caractérisé en ce que** la membrane poreuse (14) est disposée dans le support (12) et **en ce que** le support (12) comporte au moins une lumière (20), chaque lumière (20) venant au droit de chaque zone réactionnelle hydrophile (16) de la membrane poreuse.

5. Dispositif (10) de diagnostic selon l'une des précédentes revendications, **caractérisé en ce qu'**il comprend également une membrane absorbante (18) disposée sous la membrane poreuse (14).

6. Dispositif (10) de diagnostic selon l'une des précédentes revendications, **caractérisé en ce que** la zone réactionnelle hydrophile (16) de la membrane poreuse (14) comprend également des agents de capture.

7. Dispositif (10) de diagnostic selon l'une des précédentes revendications, **caractérisé en ce que** les agents de capture comprennent des antigènes de groupe/phénotype érythrocytaire.

8. Dispositif (10) de diagnostic selon la revendication 7, **caractérisé en ce que** les agents de capture sont des hématies vidées d'hémoglobine.

9. Dispositif (10) de diagnostic selon l'une des revendications 1 à 6, **caractérisé en ce que** les agents de capture sont des anticorps.

10. Dispositif (10) de diagnostic selon l'une des précédentes revendications, **caractérisé en ce que** la zone réactionnelle (16) est hydrophile sur toute l'épaisseur de la membrane poreuse.

11. Dispositif (10) de diagnostic selon l'une des précédentes revendications, **caractérisé en ce que** la zone réactionnelle (16) comporte deux zones hydrophiles (16-1, 16-2) avec un degré d'hydrophilisation plus important au centre (16-1) de la zone réactionnelle qu'en périphérie (16-2).

12. Dispositif (10) de diagnostic selon l'une des revendications 1 à 10, **caractérisé en ce que** la zone réactionnelle (16) comporte deux zones hydrophiles avec un degré d'hydrophilisation différent, l'une en surface, l'autre dans l'épaisseur.

13. Procédé de fabrication d'un dispositif (10) selon l'une des précédentes revendications, **caractérisé en ce qu'**il comprend les étapes suivantes :
- hydrophilisation d'une membrane poreuse hydrophobe (14) d'épaisseur comprise entre 0,05mm et 1,5mm et dont le diamètre des pores est compris entre 2 et 30 µm, sur au moins une zone (16) de ladite membrane, à l'aide d'au moins un détergent,
- éventuellement dépôt d'une solution d'agent de capture,
- séchage,
- assemblage de la membrane poreuse (14) et éventuellement d'une membrane absorbante (18) disposée sous la membrane poreuse (14) avec un support (12).

14. Utilisation du dispositif selon l'une des revendications 1 à 12, pour identifier et déterminer des groupes sanguins ABO, le phénotypage étendu, la recherche d'agglutinine irrégulière, la recherche d'auto-anticorps, la recherche d'antiglobuline froide et/ou la validation croisée, partir d'un échantillon de sang ou d'un de ses composants.

15. Procédé de phénotypage de groupes sanguins érythrocytaires, à partir d'un échantillon de globules rouges, permettant la détection de la présence de deux populations différentes d'antigènes, **caractérisé en ce qu'**il comprend les étapes suivantes :
- déposer une solution pour hydrater la membrane poreuse (14) au centre de la zone réactionnelle d'un dispositif (10) selon l'une des revendications 1 à 5, ladite zone réactionnelle (16) comportant deux zones hydrophiles (16-1, 16-2) avec un degré d'hydrophilisation plus important au centre (16-1) de la zone réactionnelle qu'en périphérie (16-2), et comportant au centre des agents de captures comprenant des anticorps,
- diluer les globules rouges à phénotyper dans une solution tampon,
- ajouter cette solution contenant les globules rouges à phénotyper au centre de la zone réactionnelle,
- incuber,
- déposer une solution de rinçage sur la zone réactionnelle.

16. Procédé de phénotypage de groupes sanguins érythrocytaires, à partir d'un échantillon de globules rouges, **caractérisé en ce qu'**il comprend les étapes suivantes :
- déposer une solution pour hydrater la membrane poreuse (14) au niveau de la zone réactionnelle (16) d'un dispositif (10) selon l'une des revendications 1 à 5, ladite zone réactionnelle (16) comportant une seule zone hydrophile et comportant des agents de capture comprenant des anticorps,
- éventuellement diluer les globules rouges à phénotyper dans une solution tampon,
- ajouter cette solution contenant les globules rouges à phénotyper au centre de la zone réactionnelle,
- incuber,
- déposer une solution de rinçage sur la zone réactionnelle.

17. Procédé de détection des anticorps multivalents présents dans le sang, à partir d'un échantillon de plasma, de sérum ou de sang total, **caractérisé en ce qu'**il comprend les étapes suivantes :
- déposer l'échantillon à tester sur la zone réactionnelle (16) d'un dispositif (10) selon l'une des revendications 1 à 5, ladite zone réactionnelle (16) comportant des agents de capture comprenant des antigènes,
- ajouter des hématies tests de phénotype connu, comportant les mêmes antigènes que les agents de capture,
- laisser le mélange passer à travers la zone hydrophile,
- déposer une solution de rinçage sur la zone réactionnelle.

18. Procédé de détection des anticorps anti-érythrocytaires présents dans le sang, de validation croisée ou de recherche d'auto-anticorps ou d'agglutinine froide, à partir d'un échantillon de plasma, de sérum ou de sang total, **caractérisé en ce qu'**il comprend les étapes suivantes :
- incuber l'échantillon à tester avec un tampon et des hématies tests de phénotype connu,
- ajouter à ce mélange un agent capable d'agréger les globules rouges,
- déposer le mélange sur la zone réactionnelle (16) d'un dispositif (10) selon l'une des revendications 1 à 5,
- déposer une solution contenant un agent capable d'agréger les globules rouges sur la zone réactionnelle,
- déposer un réactif de Coombs, d'antiglobuline humaine ou d'anti-complément sur la zone réactionnelle, et
- déposer une solution de rinçage sur la zone réactionnelle.

## Patentansprüche

1. In-vitro-Diagnosevorrichtung (10) zum Nachweis von mindestens einer Reaktion zwischen einem Erythrozyten-Phänotyp-Antigen und einem Antikörper, der spezifisch gegen dieses Antigen gerichtet ist, anhand einer Probe von Blut oder eines seiner Bestandteile, **dadurch gekennzeichnet, dass** sie umfasst:
- einen Träger (12), und
- eine hydrophobe poröse Membran (14) einer Dicke im Bereich zwischen 0,05 mm und 1,5 mm, und deren Porendurchmesser im Bereich zwischen 2 und 30 *µ*m liegt, wobei die Membran mindestens eine hydrophile Reaktionszone (16) umfasst, die dazu vorgesehen ist, die Probe aufzunehmen, wobei die Oberfläche der hydrophilen Reaktionszone (16) kleiner ist als die Oberfläche der hydrophoben porösen Membran (14).

2. Diagnosevorrichtung (10) nach Anspruch 1, **dadurch gekennzeichnet, dass** die hydrophile Reaktionszone (16) der porösen Membran (14) ohne Modifikation der chemischen Funktionen des porösen Substrats durch eine chemische oder physikalische Vorbehandlung der porösen Membran mit einem Detergens hydrophil gemacht wurde.

3. Diagnosevorrichtung (10) nach Anspruch 2, **dadurch gekennzeichnet, dass** das Detergens ein nichtionisches Tensid ist.

4. Diagnosevorrichtung (10) nach einem der vorstehenden Ansprüche, **dadurch gekennzeichnet, dass** die poröse Membran (14) im Träger (12) angeordnet ist, und dadurch, dass der Träger (12) mindestens eine Öffnung (20) umfasst, wobei jede Öffnung (20) gerade zu jeder hydrophilen Reaktionszone (16) der porösen Membran steht.

5. Diagnosevorrichtung (10) nach einem der vorstehenden Ansprüche, **dadurch gekennzeichnet, dass** sie ebenfalls eine absorbierende Membran (18) umfasst, die unter der porösen Membran (14) angeordnet ist.

6. Diagnosevorrichtung (10) nach einem der vorstehenden Ansprüche, **dadurch gekennzeichnet, dass** die hydrophile Reaktionszone (16) der porösen Membran (14) ebenfalls Einfangmittel umfasst.

7. Diagnosevorrichtung (10) nach einem der vorstehenden Ansprüche, **dadurch gekennzeichnet, dass** die Einfangmittel Erythrozyten-Gruppen/Phänotyp-Antigene umfassen.

8. Diagnosevorrichtung (10) nach Anspruch 7, **dadurch gekennzeichnet, dass** die Einfangmittel an Hämoglobin entleerte Erythrozyten sind.

9. Diagnosevorrichtung (10) nach einem der Ansprüche 1 bis 6, **dadurch gekennzeichnet, dass** die Einfangmittel Antikörper sind.

10. Diagnosevorrichtung (10) nach einem der vorstehenden Ansprüche, **dadurch gekennzeichnet, dass** die Reaktionszone (16) über die gesamte Dicke der porösen Membran hydrophil ist.

11. Diagnosevorrichtung (10) nach einem der vorstehenden Ansprüche, **dadurch gekennzeichnet, dass** die Reaktionszone (16) zwei hydrophile Zonen (16-1, 16-2), mit einem höheren Hydrophilisierungsgrad in der Mitte (16-1) der Reaktionszone als am Rand (16-2) umfasst.

12. Diagnosevorrichtung (10) nach einem der Ansprüche 1 bis 10, **dadurch gekennzeichnet, dass** die Reaktionszone (16) zwei hydrophile Zonen mit einem unterschiedlichen Hydrophilisierungsgrad, einem an der Oberfläche, dem anderen in der Dicke, umfasst.

13. Verfahren zum Herstellen einer Vorrichtung (10) nach einem der vorstehenden Ansprüche, **dadurch gekennzeichnet, dass** es die folgenden Schritte umfasst:
- Hydrophilisieren einer hydrophoben porösen Membran (14) einer Dicke im Bereich zwischen 0,05 mm und 1,5 mm, und deren Porendurchmesser im Bereich zwischen 2 und 30 *µ*m liegt, auf mindestens einer Zone (16) der Membran durch lokales Zugeben von mindestens einem Detergens,
- gegebenenfalls Aufbringen einer Einfangmittel-Lösung,
- Trocknen,
- Zusammenbauen der porösen Membran (14) und gegebenenfalls einer unter der porösen Membran (14) angeordneten absorbierenden Membran (18) mit einem Träger (12).

14. Verwendung der Vorrichtung nach einem der Ansprüche 1 bis 12 zum Identifizieren und Bestimmen von ABO-Blutgruppen, der erweiterten Phänotypisierung, der Suche von unregelmäßigem Agglutinin, der Suche von Autoantikörpern, der Suche von Kälte-Antiglobulin und/oder der Kreuzvalidierung anhand einer Probe von Blut oder eines seiner Bestandteile.

15. Verfahren zur Phänotypisierung von Erythrozyten-Blutgruppen anhand einer Probe von roten Blutkörperchen, das den Nachweis des Vorhandenseins von zwei unterschiedlichen Antigenpopulationen ermöglicht, **dadurch gekennzeichnet, dass** es die folgenden Schritte umfasst:
- Aufbringen einer Lösung, um die poröse Membran (14) in der Mitte der Reaktionszone einer Vorrichtung (10) nach einem der Ansprüche 1 bis 5 zu hydratisieren, wobei die Reaktionszone (16) zwei hydrophile Zonen (16-1, 16-2) mit einem höheren Hydrophilisierungsgrad in der Mitte (16-1) der Reaktionszone als am Rand (16-2) umfasst, und in der Mitte Antikörper umfassende Einfangmittel umfasst,
- Verdünnen der zu phänotypisierenden roten Blutkörperchen in einer Pufferlösung,
- Zugeben dieser Lösung, die die zu phänotypisierenden roten Blutkörperchen enthält, in die Mitte der Reaktionszone,
- Inkubieren,
- Aufbringen einer Spüllösung auf die Reaktionszone.

16. Verfahren zur Phänotypisierung von Erythrozyten-Blutgruppen anhand einer Probe von roten Blutkörperchen, **dadurch gekennzeichnet, dass** es die folgenden Schritte umfasst:
- Aufbringen einer Lösung, um die poröse Membran (14) im Bereich der Reaktionszone (16) einer Vorrichtung (10) nach einem der Ansprüche 1 bis 5 zu hydratisieren, wobei die Reaktionszone (16) eine einzige hydrophile Zone umfasst und Antikörper umfassende Einfangmittel umfasst,
- gegebenenfalls Verdünnen der zu phänotypisierenden roten Blutkörperchen in einer Pufferlösung,
- Zugeben dieser Lösung, die die zu phänotypisierenden roten Blutkörperchen enthält, in die Mitte der Reaktionszone,
- Inkubieren,
- Aufbringen einer Spüllösung auf die Reaktionszone.

17. Verfahren zum Nachweis der im Blut vorhandenen multivalenten Antikörper anhand einer Plasma-, Serum- oder Vollblutprobe, **dadurch gekennzeichnet, dass** es die folgenden Schritte umfasst:
- Aufbringen der zu testenden Probe auf die Reaktionszone (16) einer Vorrichtung (10) nach einem der Ansprüche 1 bis 5, wobei die Reaktionszone (16) Antikörper umfassende Einfangmittel umfasst,
- Zugeben von Testerythrozyten von bekanntem Phänotyp, die die gleichen Antigene umfassen wie die Einfangmittel,
- Passierenlassen der Mischung durch die hydrophile Zone,
- Aufbringen einer Spüllösung auf die Reaktionszone.

18. Verfahren zum Nachweis der im Blut vorhandenen Anti-Erythrozyten-Antikörper, zur Kreuzvalidierung oder zur Suche von Anti-Antikörpern oder Kälte-Agglutinin anhand einer Plasma-, Serum- oder Vollblutprobe, **dadurch gekennzeichnet, dass** es die folgenden Schritte umfasst:
- Inkubieren der zu testenden Probe mit einem Puffer und Testerythrozyten von bekanntem Phänotyp,
- Zugeben eines Mittels, das in der Lage ist, die roten Blutkörperchen zu aggregieren, zu dieser Mischung,
- Aufbringen der Mischung auf die Reaktionszone (16) einer Vorrichtung (10) nach einem der Ansprüche 1 bis 5,
- Aufbringen einer Lösung, die ein Mittel enthält, das in der Lage ist, die roten Blutkörperchen zu aggregieren, auf die Reaktionszone,
- Aufbringen eines Coombs-Reagenzes, von humanem Antiglobulin oder Anti-Komplement auf die Reaktionszone, und
- Aufbringen einer Spüllösung auf die Reaktionszone.

## Claims

1. An in-vitro diagnosis device (10), for detection of at least one reaction between an antigen of blood group phenotype and an antibody directed specifically against this antigen, from a sample of blood or one of its components, **characterized in that** it comprises:
- a support (12), and
- a hydrophobic porous membrane (14) of thickness between 0.05 mm and 1.5 mm and whereof the diameter of the pores is between 2 and 30 *µ*m, said membrane comprising at least one hydrophilic reactional area (16) intended to receive said sample, the surface of the hydrophilic reactional area (16) being less than the surface of the hydrophobic porous membrane (14).

2. The diagnosis device (10) according to Claim 1, **characterized in that** the hydrophilic reactional area (16) of the porous membrane (14) has been made hydrophilic with a detergent without modification of the chemical functions of the porous substrate by prior chemical or physical treatment of the porous membrane.

3. The diagnosis device (10) according to Claim 2, **characterized in that** the detergent is a non-ionic surfactant.

4. The diagnosis device (10) according to any one of the preceding claims, **characterized in that** the porous membrane (14) is arranged in the support (12) and **in that** the support (12) comprises at least one opening (20), each opening (20) corresponding to each hydrophilic reactional area (16) of the porous membrane.

5. The diagnosis device (10) according to any one of the preceding claims, **characterized in that** it also comprises an absorbent membrane (18) arranged underneath the porous membrane (14).

6. The diagnosis device (10) according to any one of the preceding claims, **characterized in that** the hydrophilic reactional area (16) of the porous membrane (14) also comprises capturing agents.

7. The diagnosis device (10) according to any one of the preceding claims, **characterized in that** the capturing agents comprise antigens of blood group/phenotype.

8. The diagnosis device (10) according to Claim 7, **characterized in that** the capturing agents are red blood cells void of haemoglobin.

9. The diagnosis device (10) according to any one of Claims 1 to 6, **characterized in that** the capturing agents are antibodies.

10. The diagnosis device (10) according to any one of the preceding claims, **characterized in that** the reactional area (16) is hydrophilic over the entire thickness of the porous membrane.

11. The diagnosis device (10) according to any one of the preceding claims, **characterized in that** the reactional area (16) comprises two hydrophilic areas (16-1, 16-2) with a greater degree of hydrophilisation at the centre (16-1) of the reactional area than at the periphery (16-2).

12. The diagnosis device (10) according to any one of Claims 1 to 10, **characterized in that** the reactional area (16) comprises two hydrophilic areas with a different degree of hydrophilisation, one at the surface, the other in the thickness.

13. A manufacturing process of a device (10) according to any one of the preceding claims, **characterized in that** it comprises the following steps:
- hydrophilisation of a hydrophobic porous membrane (14) of thickness between 0.05 mm and 1.5 mm and whereof the diameter of the pores is between 2 and 30 *µ*m, on at least one area (16) of said membrane, by adding at least one detergent locally,
- optionally depositing of a capturing agent solution,
- drying,
- assembling of the porous membrane (14) and optionally an absorbent membrane (18) arranged underneath the porous membrane (14) with a support (12).

14. Use of the device according to any one of Claims 1 to 12, to identify and determine ABO blood groups, extended phenotyping, search for irregular agglutinin, search for autoantibodies, search for cold antiglobulin and/or cross-compatibility, testing a sample of blood or one of its components.

15. A blood group typing process of, testing a sample of red blood cells , for detection of the presence of mixed field population of antigens, **characterized in that** it comprises the following steps:
- depositing a solution to hydrate the porous membrane (14) at the centre of the reactional area of a device (10) according to any one of Claims 1 to 7, said reactional area (16) comprising two hydrophilic areas (16-1, 16-2) with a degree of hydrophilisation greater at the centre (16-1) of the reactional area than at the periphery (16-2), and comprising at the centre capturing agents comprising antibodies,
- diluting the red blood cells to be typed in a buffer solution,
- adding this solution containing the red blood cells to be typed at the centre of the reactional area,
- incubating,
- depositing a rinse solution on the reactional area.

16. The blood group typing process testing a sample of red blood cells , **characterized in that** it comprises the following steps:
- depositing a solution to hydrate the porous membrane (14) at the level of the reactional area (16) of a device (10) according to any one of Claims 1 to 5, said reactional area (16) comprising a single hydrophilic area and comprising capturing agents comprising antibodies,
- optionally diluting the red blood cells o be typed in a buffer solution,
- adding this solution containing the red blood cells to be typed at the centre of the reactional area,
- incubating,
- depositing a rinse solution on the reactional area.

17. The process for detection of multivalent antibodies present in the blood, from a sample of plasma, serum or whole blood, **characterized in that** it comprises the following steps:
- depositing the sample to be tested on the reactional area (16) of a device (10) according to any one of Claims 1 to 5, said reactional area (16) including capturing agents comprising antigens,
- adding red blood cell of known phenotype, comprising the same antigens as the capturing agents,
- having the mixture pass through the hydrophilic area,
- depositing a rinse solution on the reactional area.

18. The process for detection of irregular antibodies present in the blood, cross compatibility or search for autoantibodies or cold agglutinin from a sample of plasma, serum or total blood, **characterized in that** it comprises the following steps:
- incubating the sample to be tested with a buffer, and red blood cell of known phenotype,
- adding to this mixture an agent capable of aggregating the red blood cells ,
- depositing the mixture on the reactional area (16) of a device (10) according to any one of Claims 1 to 5,
- depositing a solution containing an agent capable of aggregating the red blood cells on the reactional area,
- depositing a Coombs, human antiglobulin or anti-complement reagent on the reactional area, and
- depositing a rinse solution on the reactional area.
